# EUROPEAN PATENT APPLICATION

(11) **EP 1 707 185 A1**
(43) Date of publication of application: **04.10.2006**
(21) Application number: 06250423.8
(22) Date of filing: 26.01.2006
(51) Int. Cl.: A61K 8/37, A61Q 13/00, C11B 9/00

(54) **Solvent materials and methods for preparing fragrance compositions**

(30) Priority: 31.03.2005 US 95426
(71) Applicant: INTERNATIONAL FLAVORS & FRAGRANCES, INC., New York, NY 10019 (US)
(72) Inventor: Narula, Anubhav P.S., Hazlet, New Jersey 07730 (US); Levorse, Anthony T. Jr., Westfield, NJ 07090 (US); Huang, Jennming Steven, Marlboro, NJ 07746 (US)
(74) Representative: Lawrence, John

(57) **Abstract**

In accordance with the present invention, there are provided solvent materials for use in fragrance products, comprising from about 10 to about 12 carbon diacid esters with the *proviso* that the solvent is not dimethyl sebacate. The solvent material of the present invention may be used in combination with fragrances for use in perfumery products and other consumer products.

## Description

### Field of the Invention

The present invention is directed to solvent materials for use in fragrance compositions and to a method of making fragrance compositions comprising the solvent materials. The fragrance compositions of the present invention can be used in colognes and as well as other fragrance consumer products.

### Background of the Invention

Clear, colorless or translucent, fluids which can be suitably colored and combined with fragrances are desirable characteristics of solvent material for fragrance formulations. Solvents, such as dipropylene glycol, dipropylene glycol ethers, diethyl phthalate and isopropyl myristate, have been used in combination with fragrance materials in the prior art. There is an ongoing need for additional solvent materials which would provide the recognized advantages of prior art solvents. Ideally, such solvents would not substantially contribute to or adversely affect the odor of the fragrance composition, would be safe both environmentally and for human use, and would provide the fragrance formulation with physical and performance characteristics similar to those provided by solvents that exist in the prior art.

Accordingly, there exists a need in the art for the development of new solvents for use in fragrance compositions. It is a primary objective of this invention to provide a solvent for fragrance compositions which would be a replacement for solvents that exist in the prior art, in which, at the same time, would not adversely alter the characteristics of the fragrance compositions.

### Summary of the Invention

The present invention is directed to a solvent material comprising from about 10 to about 12 carbon diacid esters with the *proviso* the solvent is not dimethyl sebacate. The solvent material of the present material may be used in combination with fragrances for use in perfumery products and other consumer products.

In a preferred embodiment the invention the solvent materials of the present invention have the formula 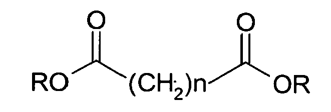 Where R and is selected from the group consisting of

CH₂CH₃; CH₂CH₂CH₂CH₃; CH₂(CH₂)₆CH₃; CH₂CH(C₂H₅)(CH₂)₃CH₃;

and
iso (C₁₃H₂₇); and n=8 or 10.

The present invention is also directed to a method for preparing a fragrance composition comprising the solvent material of the present invention and a fragrance material.

These and other embodiments of the present invention will be apparent by reading the following specification.

### Detailed Description of the Invention

The solvent material used in the practice of the present invention can be any diacid ester containing from about 10 to about 12 carbon atoms with the *proviso* that the solvent is not dimethyl sebacate.

Diacid esters suitable for use in this invention include, but are not limited to, dibutyl sebacate, diethyl sebacate, dioctyl sebacate, bis(2-ethylheptyl)dodecanedionate, bisbutyl doedcandioate and diisotridecyldodecanedioate. The preferred solvent material is characterized as being odorless, colorless, a fluid and soluble and with a flash point of greater than 225 °F.

The preferred solvent materials are listed below with the representative R and n values as set forth in the formula above:

| | | |
|---|---|---|
| Diethyl sebacate | R= CH₂CH₃ | n=8; |
| Dibutyl doedcandionate | R= CH₂-CH₂-CH₂-CH₃ | n=10; |
| Dibutyl sebacate | R= CH₂CH₂CH₂CH₃ | n=8; |
| Dioctyl sebacate | R= CH₂(CH₂)₆CH₃ | n=8 |
| and /or | CH₂CH(C₂H₅)(CH₂)₃CH₃ | |
| Bis-2-ethylhexyl dodecanedioate | R= CH₂CH(C₂H₅)(CH₂)₃CH₃ | n=10 |
| Diisotridecyldodecanedioate | R= iso(C₁₃H₂₇) | n=10. |

The use of the compositions of the present invention is widely applicable in current perfumery products, including the preparation of perfumes, colognes and toilet waters, the perfuming of personal care products such as soaps, shower gels, and hair care products as well as candles, air fresheners and cosmetic preparations. The present invention can also be used to perfume cleaning agents, such as, but not limited to detergents, dishwashing materials, scrubbing compositions, window cleaners, fabric care products such as laundry detergents, fabric softeners, bleaches, tumble dryer sheets, malodor compositions and the like. In general, the solvent material containing from about 10 to about 12 carbon diacid esters will be admixed with a fragrance to form the fragrance composition which may be used in perfumes and other consumer products.

In these preparations, the compounds of the present invention can be used alone or in combination with other perfuming compositions, solvents, adjuvants and the like. The nature and variety of the other ingredients that can also be employed are known to those with skill in the art.

Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891, the contents of which are incorporated by reference as if set forth in its entirety. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

Those with skill in the art also know that various fragrance companies provide list of fragrance materials that they supply to the industry, often these product brochures are called Perfumers Compendium. The following fragrance materials were listed in a recent version of International Flavors & Fragrances Inc. Perfumer Compendium,
4-acetyl careen ,acetyl cedrene , acetyl diisoamylene, 10-undecen-1-ol , 1-nonanal,1-decanal,2-methyl decanal; methyl octyl acetaldehyde, 2-methyl undecanal;methyl nonyl acetaldehyde, Gamma undecalactone, 1-octanal, allyl ionone, ally n-hexanoate, alpha methylene citronellal, cedryl acetate crystals,
9-decen-1-ol, 9-decen-1-yl acetate, di-n-butyl sulfide, 10-undecen-1-ol, cinnamyl nitrile, 3,7-dimethyl-6-octenyl methyl acetal
ACETYL ISOEUGENOL (2-methoxy-4-(propenyl-1)-phenyl acetate),ALLYL AMYL GLYCOLATE (glycolic acid,2-pentyloxy allyl ester), ALLYL CAPROATE (allyl n-hexanoate), ALVANONE EXTRA, ALVANONE G, AMBER ALVA ,AMBERGRIS T OLIFFAC, AMBRAIN, AMBRETTEX, AMBRETTOLIDE (oxacycloheptadec-10-en-2-one), AMBRONATE (9-decen-1-ol propionate), AMYL CINNAMIC ALCOHOL (alpha-n-amyl cinnamyl alcohol), AMYL CINNAMIC ALDEHYDE COEUR (alpha-n-amyl cinnamic aldehyde), AMYL CINNAMIC ALDEHYDE DIETHYL ACETAL (alpha-n-amyl cinnamic aldehyde diethyl acetal),AMYL CINNAMIC ALDEHYDE DIMETHYL ACETAL (alpha-n-amyl innamic aldehyde dimethyl acetal), AMYL PHENYL ACETATE (phenyl acetic acid, isoamyl ester), AMYL PROPIONATE (isoamyl propionate), AMYL SALICYLATE (pentyl ortho hydroxy benzoate), AMYL VINYL CARBINOL (1-octen-3-ol), AMYL VINYL CARBINYL ACETATE (1-octen-3-yl acetate), ANDRANE (8,9-epoxy cedrane; alpha cedrene epoxide), ANDRANE EXTRA (8,9-epoxy cedrane; alpha cedrene epoxide), ANETHOLE (para methoxy alpha phenyl propene), ANISIC ALCOHOL (para methoxy benzyl alcohol), ANISIMAL (3-phenyl-3-buten-1-yl acetate), ANISYL ACETATE (para methoxy benzyl acetate), APHERMATE (cyclohexane-1-methanol,alpha,3,3-trimethyl:formate), APO PATCHONE (para isopropyl cyclohexanol), APPLE OLIFFAC, ARBRENSE, AURALVA, BACDANOL (2-ethyl-4-(2,2,3-trimethyl-3-cyclopenten-1-yl)-2-buten-1-ol), BASIL OLIFFAC, BENZOIN COEUR, BENZOIN OLIFFAC BENZYL ACETATE, BENZYL ISOEUGENOL (isoeugenyl benzyl ether), BENZYL n-BUTYRATE (benzyl n-butanoate), BENZYL PROPIONATE (benzyl n-propanoate), BENZYL SALICYLATE (benzyl ortho hydroxy benzoate), BERGAMAL (alpha methylene citronellal),BERGAMOT SUPER OLIFFAC, BETA NAPHTHYL ISOBUTYL ETHER (naphthalene,2-isobutoxy), BIFLOR, BITTER ORANGE, BOURGEON D'AVANA,
CAMEK DH (norborane,2-acetyl-3,3-dimethyl), CAMEKOL DH (2-norbornane methanol,alpha-3,3-trimethyl), CANTHOXAL (2-methyl-3-(para-methoxy phenyl)-propanal), CARENKO (4-acetyl carene), CASHMERAN (6,7-dihydro-1,1,2,3,3-pentamethyl-4(5H)-indanone), CASSIA OLIFFAC, CASTOREUM ABSOLUTE, CEDARWOOD, CEDARWOOD OIL WHITE, CEDRACLAIRE, CEDRAMBER (cedryl methyl ether), CEDRENOL, CEDRENYL ACETATE (cedryl acetate), CEDRENYL ACETATE ESTERS (cedryl acetate), CEDRENYL FORMATE, CEDROL CRYSTALS, CELESTOLIDE (4-acetyl-6-tertiary butyl-1,1-dimethyl indane), CHAMOMILE S OLIFFAC, CHAMOMILE SUPER OLIFFAC, CINALKEX, Cinnamyl nitrile, CINNAMON BARK OLIFFAC, CINNAMYL ACETATE (3-phenyl-2-propen-1-yl acetate), CITRAL (cis/trans-3,7-dimethyl-2,6-octadien-1-al(neral/geranial), CITRAL DIMETHYL ACETAL (3,7-dimethyl-2,6-octadienal dimethyl acetal), CITRAL EXTRA (cis/trans-3,7-dimethyl-2,6-octadien-1-al (neral/geranial), CITRALEX, CITRALVA (geranonitrile,3,7-dimethyl-2,6-octadiene-1-nitrile), CITRALVA PLUS (geranonitrile,3,7-dimethyl-2,6-octadiene-1-nitrile), CITRONALVA (6-octenenitrile,3,7-dimethyl), CITRONELLOL REGULAR (3,7-dimethyl-6-octen-1-ol), CITRONELLYL ACETATE (3,7-dimethyl-6-octen-1-yl acetate), CITRONELLYL ETHYL ETHER (3,7-dimethyl-6-octenyl ethyl ether), CITRONELLYL FORMATE (3,7-dimethyl-6-octen-1-yl formate)
CITRONELLYL ISOBUTYRATE (3,7-dimethyl-6-octen-1-yl isobutyrate), CITRONELLYL LACTONE, CITRONELLYL PROPIONATE (3,7-dimethyl-6-octen-1-yl propionate), CITROTONE B (2-hexyl-1,3-dioxolane), CLARYCET (2H-pyran-4-ol,tetrahydro-4-methyl-2-propyl acetate), CLARYFOL, CLARYFOL, CLONAL (n-dodecane nitrile), CONIFERAN (ortho tertiary amyl cyclohexanyl acetate), CORTEX ALDEHYDE 50 BENZYL ALCOHOL, COSTUS OLIFFAC, COUMAREX DB, CUMINYL ACETATE (para isopropyl benzyl acetate), CUMINYL ALCOHOL (para isopropyl benzyl alcohol), CYCLABUTE (tricyclo decenyl isobutyrate), CYCLACET (tricyclo decenyl acetate), CYCLAPROP (tricyclo decenyl propionate), CYCLEMONE A (2-naphthaldehyde, 1,2,3,4,5,6,7,8-octahydro-8,8-dimethyl)
CYCLOCTAL (2-heptyl-1,3-dioxolane octanal glycol acetal), CYCLOGALBANIFF (acetic acid,(cyclohexyloxy):allyl ester), CYCLOHEXYL ETHYL ACETATE (cyclohexanethanyl acetate), CYCLOHEXYL ETHYL ALCOHOL (cyclohexanethanol), CYCLOHEXYL PROPANOL (beta methyl cyclohexane ethanol), CYCLOTROPAL (4-methyl-2-(alpha-methyl benzyl)-1,3-dioxolane), DAMASCOL, 4- (5-phenyl-5-methyl hexanone-3)
DECAVE (n-decyl vinyl ether), DECYL METHYL ETHER (n-decanyl methyl ether), DELTA DAMASCONE (Dihydro Floriffone TD), DIHYDRO CARVEOL (Carhydranol),
DIHYDRO CARVYL ACETATE (Carhydrine), DIHYDRO COUMAREX, DIHYDRO CYCLACET (tricyclo decyl acetate), DIHYDRO FLORALATE (cyclohexane methanol, 2,4-dimethyl:acetate), DIHYDRO FLORALOL (2,4-dimethyl cyclohexane methanol), DIHYDRO MYRCENOL (2,6-dimethyl-7-octen-2-ol), DIHYDRO MYRCENYL ACETATE (2,6-dimethyl-7-octen-2-yl acetate), DIHYDRO TERPINEOL (1-methyl-4-isopropyl cyclohexan-8-ol), DIHYDRO TERPINYL ACETATE, DI JASMONE, DIMETHYL BENZYL CARBINOL, DIMETHYL BENZYL CARBINYL ACETATE, DIMETHYL BENZYL CARBINYL BUTYRATE, DIMETHYL BENZYL CARBINYL ISOBUTYRATE, DIMETHYL BENZYL CARBINYL PROPIONATE, DIMETHYL CYCLORMOL (4,7-methano-1H-iden-5-ol, 3a,4,5,6,7,7a-Hexahydro-2(or 3),4-dimethyl), DIMETHYL OCTANOL (tetrahydro geraniol), DIMETHYL OCTANYL ACETATE, DIMETHYL PHENYL ETHYL CARBINOL, DIMETHYL PHENYL ETHYL CARBINYL ACETATE, DIMETHYL PHENYL ETHYL CARBINYL ISOBUTYRATE, DIMYRCENE (Alvasol), DIMYRCETOL (2,6-dimethyl-7-octen-2-ol; 2,6-dimethyl-7-octen-2-yl formate), DIOLA (methyl n-hexyl ether), DULCINYL RECRYSTALLIZED (piperonyl acetone), EPICENE GAMMA, ETHOXIFF (ethyl-3-hydroxy butyrate), ETHYL CAPROATE (ethyl hexoate), ETHYL CITRONELLYL OXALATE, ETHYL ISOVALERATE (isovaleric acid:ethyl ester), ETHYL METHYL PHENYL GLYCIDATE (glycidic acid, 3-methyl-3-phenyl:ethyl ester), ETHYL ORTHO METHOXY BENZOATE (benzoic acid,2-methoxy:ethyl ester), ETHYL-3-PHENYL GLYCIDATE (glycidic acid,3-phenyl:ethyl ester), EUGENOL PURE (phenol, 4-allyl-2-methoxy), EUGENYL ACETATE (phenol, 4-allyl-2-methoxy:acetate), EUGENYL PHENYL ACETATE (phenol, 4-allyl-2-methoxy:phenyl acetate), FIR BALSAM OLIFFAC, FLEURAMONE (n-heptyl cyclopentanone), FLORALATE (2,4-dimethyl-3-cyclohexene-1-methanyl acetate), FLORALOL (dimethyl cyclohexene methanol), FLORALOZONE (para-ethyl-alpha, alpha-dimethyl hydrocinnamaldehyde) FRAISTONE (ethyl dimethyl dioxolane acetate), FREESIA FLEURIFF, FRUCTONE (ethyl methyl dioxolane acetate), GALAXOLIDE (1,3,4,6,7,8-hexahydro-4,6,6,7,8,8-hexamethyl-cyclopenta-gamma-2-benzopyran), GALBANUM COEUR, GALBANUM RESIN OLIFFAC, GALBIFFEX R, GARDENIA FLEURIFF, GELSOL F, GELSONE (ethyl-2-hexyl acetoacetate), GERALDEHYDE (dimethyl decadienal), GERANIOL ABSOLUTE (3,7-dimethyl-2,6-octadien-1-ol), GERANIUM BOURBON SUPER OLIFFAC, GERANYL ACETATE COEUR (3,7-dimethyl-2,6-octadien-1-yl acetate), GERANYL ETHYL ETHER (3,7-dimethyl-2,6-octadien-1-ethoxy), GERANYL FORMATE (3,7-dimethyl-2,6-octadien-1-yl formate), GERANYL ISOBUTYRATE (3,7-dimethyl-2,6-octadien-1-yl-isobutyrate), GERANYL n-BUTYRATE (3,7-dimethyl-2,6-octadien-1-yl n-butyrate), GERANYL PROPIONATE (3,7-dimethyl-2,6-octadien-1-yl n-propionate), GINGER ROOT OLIFFAC, GRISALVA (naphtha [2,1-B]-furan, 3A-ethyl dodecahydro-6,6,9A-trimethyl), GUAIYL ACETATE (guai-1(5)-en-11-ol:acetate), HELIONAL (hydrocinnamaldehyde,alphamethyl-3,4-(methylenedioxy)), HERBAC (3,3-dimethyl cyclohexyl methyl ketone), HERBACET NO.1 (1-ethynyl cyclohexanyl acetate), HEXADECANOLIDE (oxacycloheptadecan-2-one), HEXALON (allyl ionone), HEXENOL (cis-3-), HEXENYL BENZOATE (cis-3-benzoic acid: 3-hexenyl ester, Z), HEXENYL SALICYLATE (cis-3-beta, gamma-hexenyl salicylate), HEXY ACETATE (acetate C-6), HEXYL CINNAMIC ALDERHYDE HEXY SALICYLATE n-(n-hexyl ortho hydroxyl benzoate), HYACINTH BODY (acetaldehyde:ethyl phenethyl acetal), HYDRATROPIC ALCOHOL COEUR (2-phenyl proply alcohol), HYDRATROPIC ALCOHOL WHITE (2-phenyl proply alcohol), HYDROXIFF, HYDROXYCITRONELLAL DIMETHYL ACETAL (octanal, 7-hydroxy-3,7-dimethyl:dimethyl acetal), HYDROXYCITRONELLAL STANDARD (octanal, 7-hydroxy-3, 7-dimethyl), HYSIMAL, INDOLAROME (indeno-m-dioxin tetrahydro), INDOLENE 50, INTRELEVEN ALDEHYDE (10-undecenal), IONONE ALPHA (3-buten-2-one,4-(2,6,6-trimethyl-2-cyclohexen-1-yl), IRITONE (trimethyl cyclohexenyl butenone), IRIVAL,ISO E SUPER (7-acetyl,1,2,3,4,5,6,7,8-octahydro-1,1,6,7-tetramethyl naphthalene), ISOAMYL SALICYLATE (2-methyl butyl salicylate), ISOBUTYL BENZYL CARBINOL (alpha-isobutyl phenethyl alcohol), ISOBUTYL QUINOLINE (quinoline, 6-secondary butyl), ISOCYCLEMONE E (2-acetonaphthone-1,2,3,4,5,6,7,8-octahydro-2, 3,8,8-tetramethyl), ISOCYCLOCITRAL (3-cyclohexene-1-carboxaldehyde,2,4,6-trimethyl), ISOCYCLOGERANIOL (trimethyl cyclohexene methanol), ISOMETHYL IONONE PURE, ALPHA (3-buten-2-one,3-methyl-4-(2,6,6-trimethyl-2-cyclohexen-1-yl)), ISOPROXEN (bicyclo [2.2.1] heptane,2-ethylidene-6-isopropoxy), JASMAL (2H-pyran-4-ol,tetrahydro-3-pentyl:acetate), JASMAL EXTRA (2H-pyran-4-ol,tetrahydro-3-pentyl:acetate), JASMELIA (2H-pyran-4-ol,3-butyltetrahydro-5-methyl:acetate), JASMIN ABSOLUTE SPECIAL OLIFFAC , JESSEMAL (2H-pyran-4-ol,3-butyltetrahydro-5-methyl:acetate), KHARISMAL (cyclopentane acetic acid,3-oxo-2-pentyl:methyl ester, cis), KOAVONE (acetyl diisoamylene), KOHINOOL (2-heptanol, 3,4,5,6,6-pentamethyl), LABDANAX, LABDANUM RESIN ABSOLUTE, LAVENDER OLIFFAC, LAVENDER SPIKE OIL, LAVONAX (4-pentenophenone), LEAF ACETAL EXTRA (acetaldehyde:ethyl-cis-3-hexenyl acetal), LEMON OIL SPANISH, LEMORALE, LEMSYN (3,7-dimethyl-2,6-octadien-1-al), LIFFAROME (cis-3-hexenyl methyl carbonate), LIME OIL, LYRAL (3-cyclohexene-1-carboxaldehyde,4-(4-hydroxy-4-methyl pentyl)), MAKROMUSKIFF, MANDARIN OLIFFACE, MARITIMA, MASSADA, MELAFLEUR, MELOZONE (4,7-methanoindan-1-carboxaldehyde,hexahydro), MERION, METHYL ANTHRANILATE (anthranilic acid: methyl ester), METHYL CINNAMIC ALDEHYDE (2-methyl-3-phenyl-2-propen-1-al), METHYL CYCLOCITRONE (trimethyl cyclohexanyl methyl ketone), METHYL EUGENOL (eugenyl methyl ester), METHYL IONONE A, gamma (3-buten-2-one,3-methyl-4-(2,6,6-trimethyl-2-cyclohexen-1-yl), METHYL LAVENDER KETONE (2-nonanone,3-(hydroxy methyl), METHYL PHENYL ETHYL ETHER (ether, methyl phenethyl), MIEL OLIFFAC, MIMOSA OLIFFAC, MUGUET ALDEHYDE (citronellyl oxyacetaldehyde), MUGUOL EXTRA (allo ocimenol), MUSK 15( cyclopentadecanone), MYRAC ALDEHYDE (isohexenyl tetrahydro benzaldehyde), MYRCENOL 50 (7-octen-2-ol,2-methyl-6-methylene), MYRCENYL ACETATE (7-octen-2-ol,2-methyl-6-methylene:acetate), MYRRH COEUR, MYSORIFF, NEOPROXEN (bicyclo[2.2.2]hept, 2-eth-5(or 6)-methoxy+tricyclo[2.2.1.0(2,6)]hept, 1-eth-3-methoxy), NERGER FORMATE (2,6-octadien-1-ol,3,7-dimethyl:formate), NEROL COEUR (2,6-octadien-1-ol, 3,7-dimethyl), NEROLI OLIFFAC, NERYL ACETATE (2,6-octadien-1-ol, 3,7-dimethyl:acetate), NONYL ACETATE (acetate C-9, nonyl alcohol: acetate), OAKMOSS OLIFAC 924, OCIMENE (3,7-dimethyl-1,3,6-octatriene), OCIMENOL (5,7-octadien-2-ol, 2,6-dimethyl), OCIMENYL ACETATE (5,7-octadien-2-ol, 2,6-dimethyl:acetate), OCTACETAL, OLIBANUM COUER 50, OPOPONAX RESIN, ORAFLEUR, ORANGE FLOWER ETHER (ether, p-menth-1-en-8-yl methyl), ORIVONE (para tertiary amyl cyclohexanone), OSMANTHUS FLEURIFF, OXASPIRANE (1-oxaspiro [5.5] undecane, 4-methylene), OXYPHENYLON (hydroxyl phenyl butanone), PAMPLEFLEUR (2-methyl-4-phenyl-1-pentanol),PARA CRESYL CAPRYLATE (para tolyl octanoate), PARA CRESYL ISOBUTYRATE (para tolyl isobutyrate), PARA METHYL BENZYL ACETATE (para tolyl acetate), PARA METHYL DIMETHYL BENZYL CARBINOL, (benzene ethanol, alpha, alpha-4-trimethyl), PARA METHYL QUINOLINE (6-methyl quinoline), PATCHOMINT (delta-2,beta-norbornane ethanol, 3,3-dimethyl), PATCHONE (para tertiary butyl cyclohexanol), PATCHOULI, PEACH ALDEHYDE COUER (aldehyde C-14, gamma undecalactone), PEOMOSA, PERU BALSAM OLIFFAC, PETITGRAIN ABSOLUTE, PHENAFLEUR (phenyl ethyl cyclohexyl ether), PHENOXANOL (1-pentanol, 3-methyl-5-phenyl), HENOXYETHYL ISOBUTYRATE (isobutyrate, 2-phenoxyethyl), PHENOXYETHYL PROPIONATE (propionate, 2-phenoxyethyl), PHENYL ETHYL ACETATE (beta phenyl ethyl acetate) PHENYL ETHYL BENZOATE (beta phenyl ethyl benzoate), PHENYL ETHYL CINNAMATE (beta phenyl ethyl cinnamate), PHENYL ETHYL FORMATE (beta phenyl ethyl formate), PHENYL ETHYL ISOBUTYRATE (beta phenyl ethyl isobutyrate), PHENYL ETHYL METHYL ETHYL CARBINOL (beta phenyl ethyl methyl ethyl carbinol), PHENYL ETHYL PROPIONATE (beta phenyl ethyl propionate), PHENYL ETHYL SALICYLATE (beta phenyl ethyl salicylate), PHENYL PROPYL ALDEHYDE (dihydro cinnamic aldehyde),PICOMATE (2H-2, 4A-methanonaphthalen-8(5H)-one, hexahydro-1,1,5,5-tetramethyl), PICONIA (2H-2,4A-methanonaphthalen-8(5H)-one,hexahydro-1, 1,5,5-tetramethyl), PINENE alpha, PINOACETALDEHYDE (2-norpinene-2-propionaldehyde, 6,6-dimethyl), RECYCLEMONE B (3-cyclohexene-1-carboxaldehyde, 1-methyl-4-(4-methyl-3-pentenyl), PRENYL ACETATE (2-buten-1-ol, 3-methyl:acetate), PROFLORA (prenyl benzoate), PSEUDO LINALYL ACETATE, RESEDA BODY (4,4,6-trimethyl-2-benzyl-1,3-dioxane), RHODINYL ACETATE, ROSALVA (9-decen-1-ol), ROSAMUSK (cyclohexane-1-methanol,alpha,3,3-trimethyl:acetate), ROSE ABSOLUTE , ROSE TENACE, ROSEATE (9-decen-1-yl acetate), ROSEMAREL (bicyclo [3.1.1] heptane, 6,6-dimethyl-2-methylene), ROSEMARIFF, ROSEMARY OIL SPANISH, SAGE CLARY OLIFFAC, SALVONE 7, SANDALWOOD OLIFFAC, SANTALOL (2-penten-1-ol,5-(2,3-dimthyl tricyclo[2.2.1.0(2,6)]hept-3-yl)-2-methyl), SHANGRALIDE, SPIRODECANE (1,5-dioxaspiro[5.5] undecane, 2-methyl), STRAWBERRIFF (2-methyl-2-pentenoic acid), STRAWBERRY OLIFFAC, STYRAX ALVA ESSENCE, STYRAX COEUR,SUEDERAL HT, SUPAFLEUR, SUPALANG, SYVERTAL (2-ethyl hexanal: cycloglycol acetal), TERPINEOL (3-cyclohexene-1-methanol, alpha, alpha, 4-trimethyl), TERPINEOL PFG (3-cyclohexene-1-methanol, alpha, alpha, 4 trimethyl), TERPINYL ACETATE (3-cyclohexene-1-methanol,alpha, alpha, 4-trimethyl:acetate), TETRAHYDRO MUGUOL (3-octanol, 3,7-dimethyl/2-octanol, 2,6-dimethyl), TETRAHYDRO MUGUOL COEUR (3-octanol, 3,7-dimethyl/2-octanol, 2,6-dimethyl), TETRAHYDRO MUGYL ACETATE (3-octanol, 3,7-dimethyl/2-octanol, 2,6-dimethyl:acetate), TETRAHYDRO MYRCENOL (2-octanol, 2,6-dimethyl), TETRAMERAN (6,10-dimethyl-9-undecen-2-one), TIMBERIFF, TOBACAROL (5,6-epoxy-2,6,10,10-tetramethyl bicyclo[7.2.0]undecane, TONQUITONE, TRANS DECAHYDRO BETA NAPHTHOL (2-naphthol,decahydro), TRANS DECAHYDRO BETA NAPHTHYL ISOBUTYRATE (2-naphthol, decahydro:isobutyrate),TRIMOFIX "O" (2,5,10-trimethyl-2,5,9-cyclododecatrien-1-yl methyl ketone & isomers),TRIPLAL (3-cyclohexene-1-carboxaldehyde, dimethyl), TUBEROSE OLIFFAC, VAMBA, VANDOR B (3,5,5-trimethyl hexanal), VANILLA ABSOLUTE, VANORIS, VERAFLOR, VERAMOSS (beta-resorcyclic acid, 3,6-dimethyl:methyl ester), VERDOL (ortho tertiary butyl cyclohexanol), VERDONE (ortho tertiary butyl cyclohexanone), VERDOX (ortho tertiary butyl cyclohexanyl acetate), VERTENEX (para tertiary butyl cyclohexanyl acetate), VERTENEX INTERCIS, VERTOFIX COEUR, and VETACETEX.

Olfactory effective amount is understood to mean the amount of compound in perfume compositions the individual component will contribute to its particular olfactory characteristics, but the olfactory effect of the perfume composition will be the sum of the effects of each of the perfumes or fragrance ingredients.

The range of the solvent is in the order of from about 0.1 to about 99 weight percent, preferably for 10 to about 90 weight percent, more preferably of from about 10 to about 30 weight percent, and in a highly preferred embodiment of from about 10 to about 25 weight percent. The level of fragrance employed in the consumer product varies from about 0.005 to about 25 weight percent, preferably from about 0.5 to about 15 and most preferably from about 1 to about 7 weight percent. In addition to the fragrances and the solvents other agents can be used in conjunction with the composition.

The solvents of the present invention are typically admixed with fragrance materials to form a fragrance concentrate in the range of from about 5 to about 50 weight percent solvent, preferably from about 10 to about 30 and most preferably from about 15 to about 25 weight percent. The concentrated fragrance material is later diluted with additional solvent so that the solvent may be present in an amount up to 90 weight percent for final packaging of a consumer product such as a fine fragrance or cologne.

Well known materials such as surfactants, emulsifiers, polymers to encapsulate the fragrance can also be employed without departing from the scope of the present invention. Those with skill in the art will be able to employ the desired level of the compounds of the invention to provide the desired fragrance and intensity. The fragrance solvent materials of the present invention can be used in combination with other solvent materials, such as dipropylene glycol, dipropylene glycol ethers, diethyl phthalate and isopropyl myristate and the like.

The present invention possesses a number of desirable characteristics for use as solvents for fragrance materials. The solvents of the present invention are generally water insoluble, meaning that less than 0.1 percent water is soluble in these materials. The solvent materials possess solubility with fragrance materials, good color properties meaning does not add color to the product, are stable which means that they are unlikely to react with the fragrance materials and are suitable for repeated contact with human skin. A key advantage of the fragrance materials of the present invention is that they possess low odor levels. This is important in that a perfumer would prefer to use a material that does not contribute an odor to the final fragrance product.

Another advantage of the solvent material of the present invention is the reduced flash point of the fragrance composition. Generally, the fragrance compositions will have a flash point that is safe for the materials to be used is greater than 225 °F.

The solvent materials of the present invention are known in the art. For example, both bis-2-ethylhexyl dodecanedioate and diisotridecyldodecanedioate are commercially available for Invista™ (UK) Ltd., Wilton Site, TS10 4XY Redcar, United Kingdom. These materials are also commercially available from Aldrich Chemical Company. Further U.S. Patent Application 2003/0224030 discloses the use of dibutyl sebacate as a plasticizer in a method of reducing airborne particulates from a surface in combination with a particulate-controlling polymer and an aqueous carrier. The patent states that the plasticizers allow for incorporation of a much wider range of particulate-controlling polymers in the non-liquid compositions.

The following are provided as specific embodiments of the present invention. Other modifications of this invention will be readily apparent to those skilled in the art. Such modifications are understood to be within the scope of this invention. As used herein all percentages are weight percent unless otherwise noted, ppm is understood to mean parts per million; mm is understood to be millimeters, ml is understood to be milliliters, Bp is understood to be boiling point. IFF as used in the examples is understood to mean International Flavors & Fragrances Inc., New York, NY, USA.

In order to demonstrate the invention, the following examples were conducted. All U.S. Patent and Patent applications referenced herein are hereby incorporated by reference as if set forth in their entirety. The following disclosures are provided to exemplify the present invention.

Unless noted to the contrary all weights are weight percent. Upon review of the foregoing, numerous adaptations, modifications and alterations will occur to the reviewer. These adaptations, modifications, and alterations will all be within the spirit of the invention. Accordingly, reference should be made to the appended claims in order to ascertain the scope of the present invention.

### EXAMPLES

The following fragrance materials were evaluated for their ability to act as a solvent for the following fragrance materials (all IFF fragrance materials unless noted otherwise).

The test was conducted by attempting to make a 10% solution in each of the following solvents:
bis-2-ethylhexyl dodecanedioate and diisotridecyldodecanedioate
   Aldehyde AA Tripal
   Aldehyde C-10
   Amaryllide FF
   Ambrettolide
   Calone
   Canthoxal
   Caryophyllene
   Cedramber
   Galaxolide White

All of the above fragrance materials were soluble in both solvents.

One fragrance material Myrrh Coeur (50% solution) was not readily soluble in either solvent.

This example indicates that the solvents are well suited to be used as solvent for fragrance materials.

## Claims

1. A solvent material comprising from about 10 to about 12 carbon diacid esters with the *proviso* that said solvent material is not dimethyl sebacate.

2. The solvent material of claim 1 wherein the solvent material is selected from the group consisting of dibutyl sebacate, diethyl sebacate, dioctyl sebacate, bis(2-ethylheptyl)dodecanedioate, bisbutyl doedcandioate and diisotridecyldodecanedioate.

3. A fragrance composition that comprises a fragrance and a solvent material wherein the solvent material comprises from about 10 to about 12 carbon diacid esters with the *proviso* that said solvent material is not dimethyl sebacate.

4. The fragrance composition of claim 3 wherein the solvent material is selected from the group consisting of dibutyl sebacate, diethyl sebacate, dioctyl sebacate, bis(2-ethylheptyl)dodecanedioate, bisbutyl doedcandioate and diisotridecyldodecanedioate.

5. The fragrance composition of claim 3 or claim 4 wherein the fragrance composition is incorporated into a product selected from the group consisting of perfumes, colognes, candles, toilet waters, cosmetic products, personal care products, fabric care products, cleaning products, air fresheners, soaps, detergents, dishwashing compositions, scrubbing compounds and window cleaners.

6. The fragrance composition according to claim 4 or claim 5, wherein the solvent is present at a level from about 10 to about 90 weight %.

7. The fragrance composition according to claim 4 or claim 5, wherein the solvent is present at a level from about 10 to about 25 weight %.

8. A method of making a fragrance composition comprising:
providing a solvent containing from about 10 to about 12 carbon diacid esters with the *proviso* that said solvent material is not dimethyl sebacate;
providing a fragrance;
admixing the solvent and the fragrance; and
forming the fragrance composition.

9. A method of making a fragrance composition according to claim 8 wherein the solvent is selected from the group consisting of dibutyl sebacate, diethyl sebacate, dioctyl sebacate, bis(2-ethylheptyl)dodecanedioate, bisbutyl doedcandioate and diisotridecyldodecanedioate.

10. The method of claim 9, wherein the solvent is present at a level from about 0.1 to about 99 weight %.

11. The method of claim 9, wherein the solvent is present at a level from about 10 to about 25 weight %.

12. The method of any one of claims 9 to 11 wherein the fragrance composition is incorporated into a product selected from the group consisting of perfumes, colognes, toilet waters, cosmetic products, personal care products, fabric care products, cleaning products and air fresheners.

13. The method of claim 12 wherein the cleaning product is selected from the group consisting of detergents, dishwashing compositions, scrubbing compounds and window cleaners.
